# EUROPEAN PATENT APPLICATION

(11) **EP 4 053 279 A1**
(43) Date of publication of application: **07.09.2022**
(21) Application number: 19949535.9
(22) Date of filing: 17.12.2019
(51) Int. Cl.: C12N 15/57, C12R 1/72, C12P 21/06, C12N 1/19, C12R 1/07

(54) **CANDIDA UTILIS DOUBLE GENE CO-EXPRESSION STRAIN THAT HYDROLYZES PROTEIN COMPONENTS IN FOOD WASTE AND CONSTRUCTION METHOD THEREFOR**

(30) Priority: 18.10.2019 CN 201910998677
(71) Applicant: Guangdong Recyclean Low-Carbon Technology Co., Ltd, Guangzhou, Guangdong 510660 (CN)
(72) Inventor: LIU, Zehuan, Guangzhou, Guangdong 510660 (CN); LIN, Jianghai, Guangzhou, Guangdong 510660 (CN); SUN, Tao, Guangzhou, Guangdong 510660 (CN)
(74) Representative: Puschmann Borchert Kaiser Klettner Patentanwälte Partnerschaft mbB
(86) International application number: PCT/CN2019/125985
(87) International publication number: WO 2021/072963

(57) **Abstract**

The present invention relates to the fields of genetic engineering and fermentation engineering, and provides a *Candida utilis* double gene co-expression strain for hydrolyzing protein components in kitchen waste, in which the *Candida utilis* double gene co-expression strain is constructed by integrating carboxypeptidases and endoprotease genes through a *Candida utilis* expression vector onto a *Candida utilis* genome. The present invention further provides a *Candida utilis* double gene co-expression strain capable of degrading kitchen waste, specifically degrading the protein components in the kitchen waste, and decomposing and transforming the proteins into small peptides and amino acids.

## Description

### TECHNICAL FIELD

The present invention relates to the fields of genetic engineering and fermentation engineering, and more particularly to a *Candida utilis* double gene co-expression strain for hydrolyzing protein components in kitchen waste and a construction method thereof.

### BACKGROUND ART

Protein components in kitchen waste are the main components in the kitchen waste other than carbohydrates and grease. After the kitchen waste is fermented, microbes substantially consume carbohydrate materials in the kitchen waste with more protein materials as residuals. Thus, protein components in the kitchen waste are liable to cause environmental pollution if discharged directly into the environment. However, conversion and utilization of proteins in the kitchen waste is an intractable problem to tackled.

Microbial fermentation receives attention of domestic and overseas industries, as fermentation techniques are becoming an effective way to utilize kitchen wastes comprehensively to achieve resource-saving and environment-friendly purposes indeed. Generally, fermentation residual liquid refers to a general term of waste residues or waste liquid produced after microbial fermentation of raw materials in industrial production applications. Residual liquid after kitchen waste fermentation refers to waste not utilized by microbes and obtained from a series of process flows on kitchen waste materials, such as degreasing, fermentation, and fermentation product extraction. After degreasing and fermentation in the above process, original grease and carbohydrate compounds in the kitchen waste are substantially utilized, while only a small portion of protein compounds in the kitchen waste are utilized by microbial growth, and most of the proteins remain in the fermentation residual liquid in the form of waste. With further research and development of kitchen waste fermentation in the industry, more and more residual liquid must be produced after fermentation. If discharged at will without effective treatment, large amounts of protein organics in the residual liquid may not only cause substantial reproduction of toxic and harmful microbes, but may also cause serious harm to the environment. Therefore, it is necessary to take effective treatment methods to treat the kitchen fermentation residual liquid, and the key to the treatment is to further utilize the organics therein. A feasible method is to decompose and transform a large amount of proteins into small peptides and amino acids, which not only solves a problem of remaining protein contamination, but also has some economic values since small peptides and amino acids are raw materials for pharmacy, biological feed, and organic fertilizer.

Compared to traditional fertilizers, amino acid fertilizer supplies higher fertility with quick absorption, and also can effectively avoid environmental pollution as an environment-friendly fertilizer. If the protein components in the kitchen waste can be transformed into small peptides or amino acids, raw materials can be provided for production of amino acid fertilizers. In this way, the problem of remaining protein contamination after kitchen waste fermentation can be solved, and considerable economic benefit can also be produced, which has great significance to the society.

Protein components in the kitchen waste are complicated, and thus a single protease often does not have a good effect, synergism of multiple enzymes is required, and ratios between different enzymes also greatly influence hydrolysis effects.

### SUMMARY OF THE INVENTION

The present invention aims to overcome at least one of the deficiencies in the prior art described above, and provide a *Candida utilis* double gene co-expression strain for hydrolyzing protein components in kitchen waste and a construction method thereof, which is capable of protein degradation in fermentation liquid of kitchen waste.

Provided is a *Candida utilis* double gene co-expression strain for hydrolyzing protein components in kitchen waste according to the present invention, in which the *Candida utilis* double gene co-expression strain is constructed by integrating carboxypeptidases and endoprotease genes through a *Candida utilis* expression vector onto a *Candida utilis* genome.

The present invention focuses on constructing a *Candida utilis* double gene co-expression strain capable of degrading kitchen waste, transforming the carboxypeptidases and the endoprotease genes into the *Candida utilis* for secretory expression, in which a selected tool is a *Candida utilis* expression vector.

As an exonuclease, a carboxypeptidase can selectively hydrolyze a C end of the protein to produce a corresponding amino acid protease, and carboxypeptidase enzyme molecules in zymogen forms can be found in a variety of organisms. The carboxypeptidase is involved in metabolism of multiple organs in the body. Moreover, in medical applications, undesirable substances produced in many organisms depend on hydrolysis of the carboxypeptidase. Nowadays, a major source of carboxypeptidase production is carboxypeptidases generated by metabolism of microbes themselves or carboxypeptidases taking microbes as host overexpression.

The endoprotease can break a polypeptide chain of protein molecules from the middle into segments or tens of segments. The role of digestion, clotting, complement activation physiological functions and other aspects, which are widely present in an organism, is to produce nucleophilic reaction between an activated hydroxy group and atoms of a peptide bond by identifying a corresponding amino acid residue chain.

*Candida utilis* has been widely used in production and life, and can be regarded as a safe microbial strain via certification of the US Food and Drug Administration. The *Candida utilis* has a series of excellent properties: having simple culturing conditions, in which the *Candida utilis* can be grown under some simple culturing conditions; having a pentose and hexose co-transport system capable of being shared by pentose and hexose, which broadens application range; capable of being applied to expressions of various target proteins due to failure of extracellular protease secretion, and the *Candida utilis* itself, as a biosafety organism, requiring only a simple separation and purification program to separate target products, thereby greatly saving production costs.

The *Candida utilis* expression vector is preferably a *Candida utilis* polygenic co-expression vector.

The *Candida utilis* expression vector is preferably GAP-pepA-TEF1-pepF.

A construction method of a *Candida utilis* double gene co-expression strain for hydrolyzing protein components in kitchen waste, including the following steps:
S1: ligating an endoprotease gene pepA to an expression vector pcGAPGA;
S2: ligating a carboxypeptidase gene pepF to an expression vector pcTEF1GA;
S3: obtaining a carboxypeptidase gene pepF expression cassette;
S4: linearizing an expression vector pcGAPGA-pepA;
S5: ligating the above carboxypeptidase gene expression cassette to an expression vector pcGAPGA-pepA to obtain a co-expression vector containing both carboxypeptidase and endoprotease gene; and
S6: cutting and linearizing the recombinant double gene co-expression vector obtained from the above construction by restriction endonuclease *Sac*I, transforming the *Candida utilis*, and constructing a genetic recombinant *Candida utilis* double gene co-expression strain.

The construction method further includes hydrolyzing protein components in kitchen waste by the recombinant *Candida utilis* double gene co-expression strain.

The transformation described in step S6 refers to transformation using an electrotransformation method, a freezing method, or a chemical reagent method.

The endoprotease in step S1 is endoprotease pepA in Aspergillus niger, and the carboxypeptidase in step S2 is carboxypeptidase pepF in Aspergillus niger.

Compared to the prior art, the present invention has the following beneficial effects. A large nember of nutrient substances are contained in the kitchen waste, and original grease and carbohydrate compounds in the kitchen waste are substantially removed after degreasing and fermentation, while only a small portion of protein compounds in the kitchen waste are utilized by microbial growth, and most of the proteins remain in the fermentation residual liquid in the form of waste. In view of this, the present invention provides a *Candida utilis* double gene co-expression strain capable of degrading kitchen waste, treating fermentation residual liquid of kitchen waste, further utilizing organics therein, and transforming a substantial amount of proteins into small peptides and amino acids. The present invention adjusts ratios between different proteases, uses synergism between proteases, seeks an optimal protease ratio, and achieves optimal efficiency to obtain maximum amino acid production.

### BRIEF DESCRIPTION OF THE DRAWINGS

FIG. 1 shows ligating an endoprotease gene pepA and a vector pMD19-T, transforming bacteria solution DH5a, and performing PCR identification.
FIG. 2 shows ligating a carboxypeptidase gene pepF and the vector pMD19-T, transforming the bacteria solution DH5a, and performing the PCR identification.
FIG. 3 shows ligating the endoprotease gene pepA and an expression vector pcGAPGA, and performing positive identification.
FIG. 4 shows ligating the carboxypeptidase gene pepF and an expression vector pcTEF1GA, and performing positive identification.
FIG. 5 is a pepF expression cassette gene.
FIG. 6 shows linearizing a pcGAPGA-pepA expression vector *Bam*HI.
FIG. 7 shows performing inverse PCR on the linearized pcGAPGA-pepA.
FIG. 8 shows ligating a pepF expression cassette and a pcGAPGA-pepA vector after inverse PCR, transforming the DH5a, and performing the positive identification.
FIG. 9 shows linearizing a double gene expression vector.
FIG. 10 shows electrotransforming *Candida utilis* and performing positive identification.
FIG. 11 is a growth curve for recombinant *Candida utilis* TGCE protease activity.
FIG. 12 shows a recombinant plasmid construction process of pcGAPGA-pepA and pcTEF1GA-pepF.
FIG. 13 is a growth curve for recombinant *Candida utilis* scTEF1-pepF protease activity.
FIG. 14 is a growth curve for recombinant *Candida utilis* cuGAP-pepA protease activity.
FIG. 15 is a construction process of double gene co-expression vector GAP-pepA-TEF1-pepF.
FIG. 16 is a construction process of triple-gene co-expression vector GAP-pepA-TEF1-YPDF-HXT7-pepF.
FIG. 17 is a growth curve for triple-gene co-expression recombinant *Candida utilis* protease activity.

### DETAILED DESCRIPTION

The drawings are for illustrative purposes only and are not to be construed as limiting the present invention. For better explanation of the following embodiments, some components in the drawings may be omitted, enlarged, or reduced, and sizes of these components do not represent sizes of actual products. For those skilled in the art, it will be understood that some known structures and descriptions thereof in the drawings may be omitted.

### Embodiment

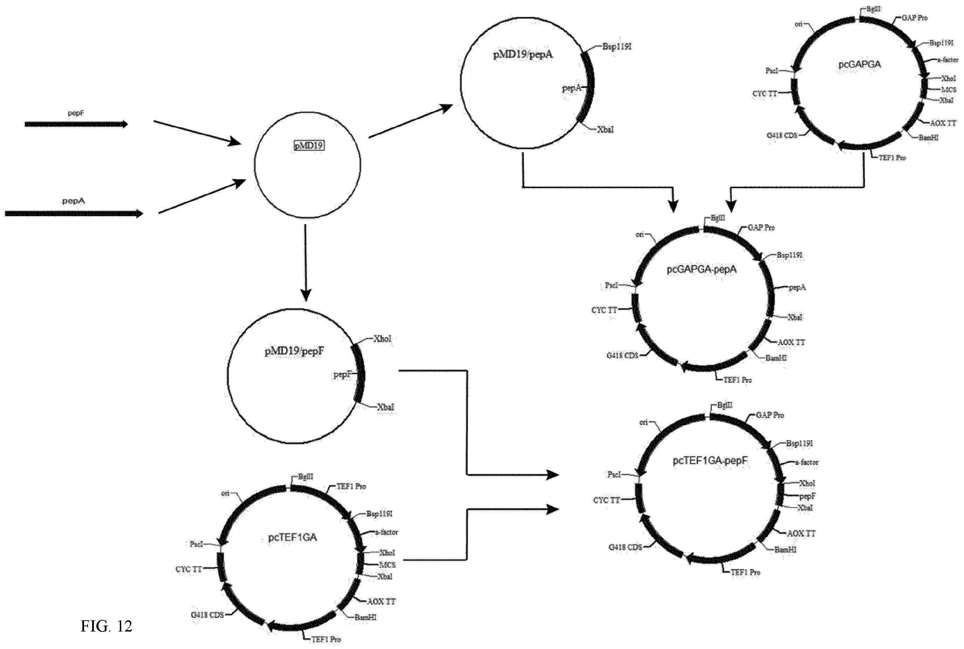

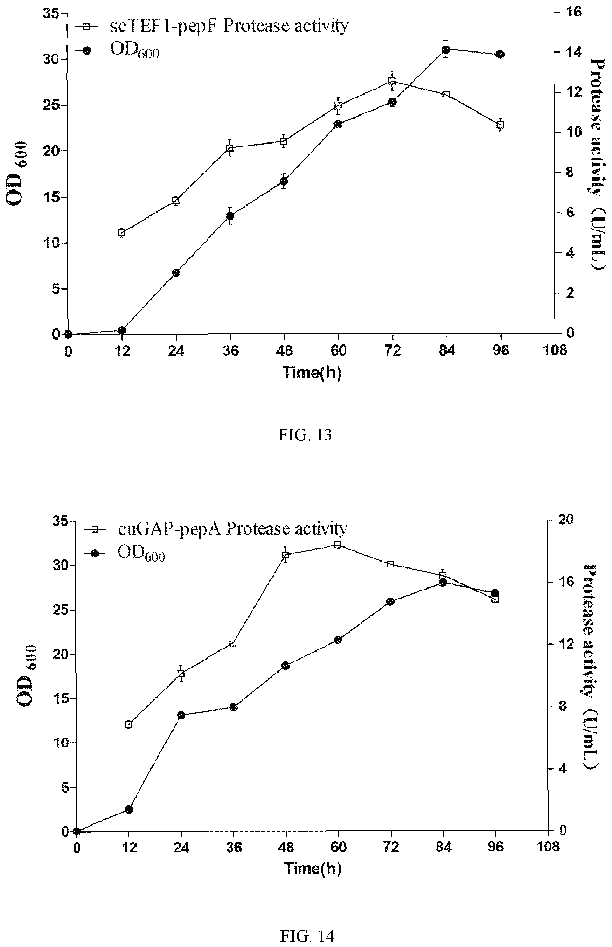

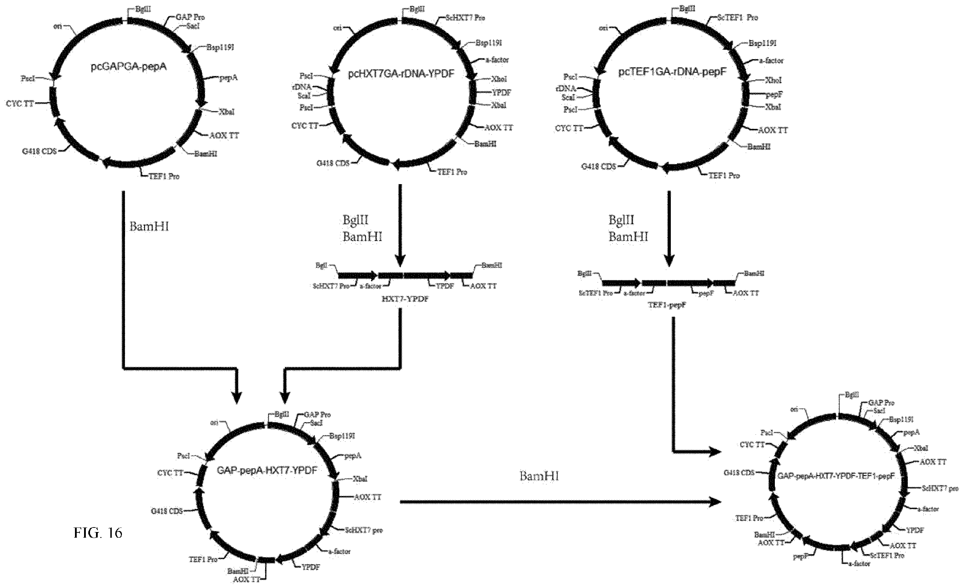

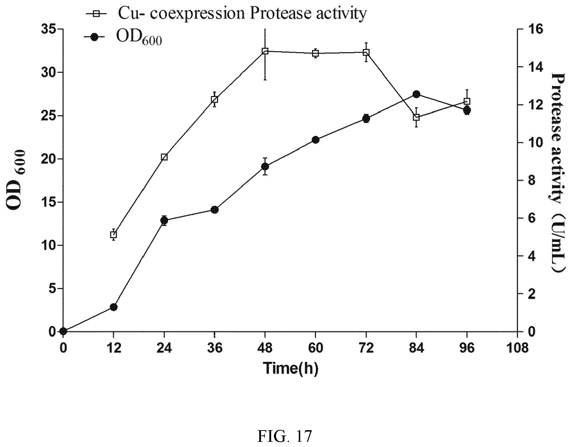

A pMD19-X recombinant plasmid was obtained by recombining an amplified target gene and a pMD19-T vector, that is, a recombinant plasmid containing a target gene X, in which X represents a pepA gene or a pepF gene. Digestion was performed on the recombinant plasmids containing pepA or pepF, while digestion was respectively performed on *Candida utilis* expression vectors pcGAPGA and pcTEF1GA. A pcGAPGA-pepA recombinant plasmid was obtained by ligating the expression vector pcGAPGA and the target gene pepA that were obtained after digestion. A pcTEF1GA-pepF recombinant plasmid was obtained by ligating the expression vector pcTEF1GA and the target gene pepF that were obtained after digestion. FIG. 12 shows structures of pcGAPGA and pcTEF1GA and a recombinant plasmid construction process. A recombinant *Candida utilis* containing carboxypeptidase pepF single gene was obtained after electrotransforming the pcTEF1GA-pepF recombinant plasmid into the *Candida utilis.* After being performed with biomass and protease activity determination, the recombinant strain was named scTEF1-pepF, and a graph drawn for the determination is shown in FIG. 13. A recombinant *Candida utilis* containing endoprotease pepA single gene was obtained after electrotransforming the pcGAPGA-pepA recombinant plasmid into the *Candida utilis.* After being performed with biomass and protease activity determination, the recombinant strain was named cuGAP-pepA, and a graph drawn for the determination is shown in FIG. 14. Based on the obtained expression vector pcGAPGA-pepA, an aminopeptidase gene expression cassette YPDF and a carboxypeptidase gene expression cassette pepF were obtained respectively; the YPDF gene expression cassette and the pcGAPGA-pepA expression vector were ligated and transformed to obtain an expression vector containing both aminopeptidase and endoprotease gene, which was named GAP-pepA-HXT7-YPDF; the vector and the pepF gene expression cassette were ligated and transformed to obtain a triple-gene expression vector containing aminopeptidase, carboxypeptidase and endoprotease, which was named GAP-pepA-TEF1-YPDF-HXT7-pepF. Specific construction process and structure are shown in FIG. 16. After electrotransforming the *Candida utilis* for biomass and protease activity determination, a graph is drawn and shown in FIG. 17.

### 1. Respective ligation between the endoprotease gene pepA and the carboxypeptidase gene pepF with expression vectors pcGAPGA and pcTEF1GA

(1) Endoprotease pepA was ligated to expression vector pcGAPGA.

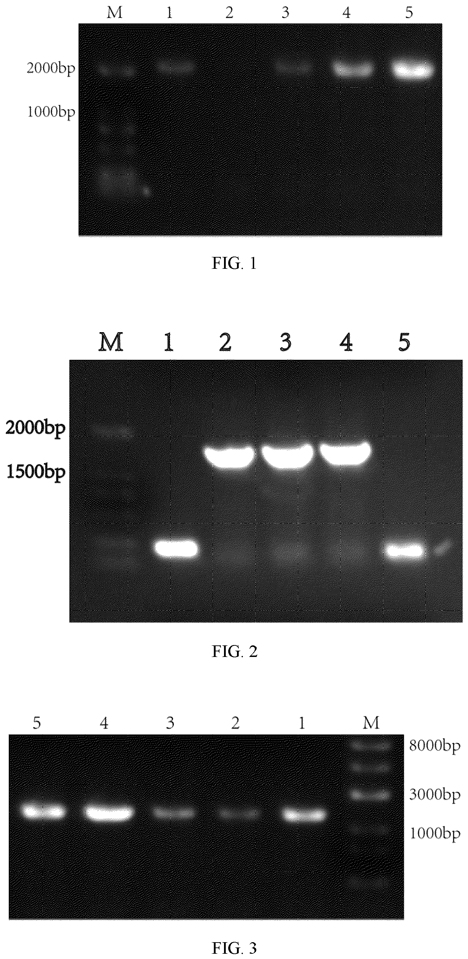

A genome was extracted from Aspergillus niger, and a pepA fragment was obtained using a primer pepA F/R and PCR, in which the sequence of pepA fragment is shown as the SEQ ID No. 2 sequence in a sequence table. After ligation of the obtained segment and the pMD19-T vector, heat-shock transformation was performed on the DH5a E. coli for bacteria solution PCR identification, and the result is shown in FIG. 1.

The pMD19-T vector and the pcGAPGA expression vector, which were ligated with the pepA gene, were respectively double-digested with two restriction endonucleases *Xho*I and *Xba*I, and target fragments were recovered.

After ligation of the double-digested pepA gene and the double-digested pcGAPGA expression vector using a DNA ligase, the recombinant expression vector pcGAPGA-pepA was constructed, heat-shock transformation was performed on the DH5a E. coli for bacteria solution PCR identification, and the result is shown in FIG. 3.

(2) The carboxypeptidase pepF was ligated to the expression vector pcTEF1GA.

A genome was extracted from Aspergillus niger, and a pepF fragment was obtained using a primer pepF F1-R4 and overlap extension PCR, in which the sequence of pepF fragment is shown as the SEQ ID No. 1 sequence in a sequence table. After ligation of the obtained segment and the pMD19-T vector, heat-shock transformation was performed on the DH5a E. coli for bacteria solution PCR identification, and the result is shown in FIG. 2.

The pMD19-T vector and the pcTEF1GA expression vector, which were ligated with the pepF gene, were respectively double-digested with two restriction endonucleases *Xho*I and *Xba*I, and target fragments were recovered.

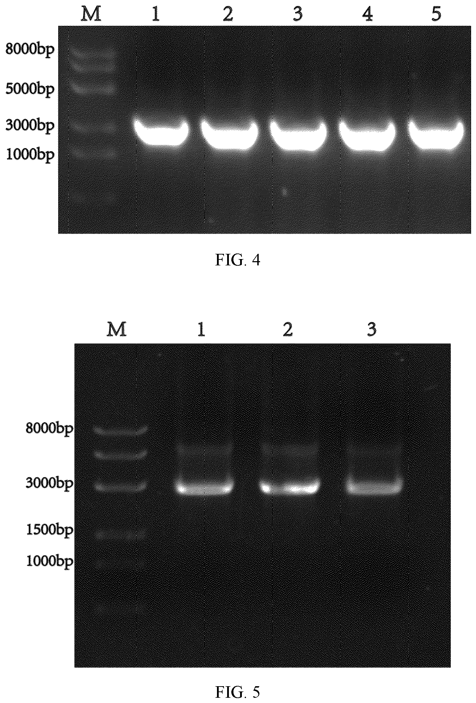

After ligation of the double-digested pepF gene and the double-digested pcTEF1GA expression vector using a DNA ligase, the recombinant expression vector pcTEF1GA-pepF was constructed, heat-shock transformation was performed on the DH5a E. coli for bacteria solution PCR identification, and the result is shown in FIG. 4.

### 2. Acquisition of a pepF expression cassette

Plasmids were extracted from DH5a E. coli containing the recombinant expression vector pcTEF1GA-pepF plasmid. A pepF expression cassette of a pepF gene fragment containing a TEF1 promoter was obtained taking recombinant expression vector pcTEF1GA-pepF plasmid as a template and using a primer pepF casF/R and PCR amplification, and the result is shown in FIG. 5.

### 3. Linearization of recombinant expression vector pcGAPGA-pepA

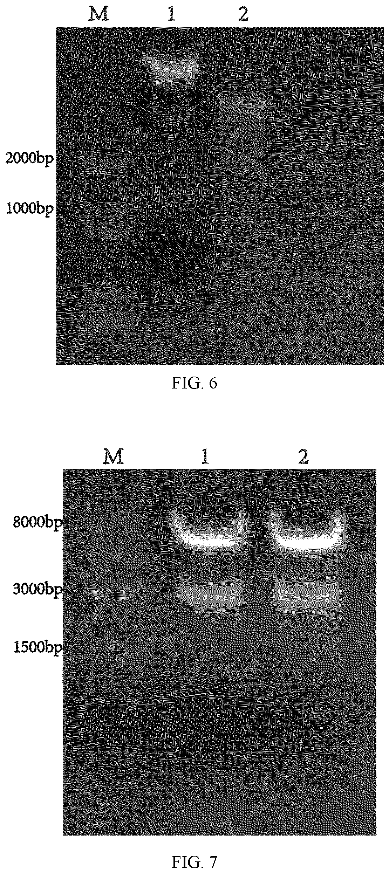

Plasmids were extracted from DH5a E. coli containing the recombinant expression vector pcGAPGA-pepA plasmid. Linearized treatment was performed on the recombinant vector taking recombinant expression vector pcGAPGA-pepA plasmid as a template and using restriction endonuclease *Bam*HI. The result is shown in FIG. 6.

To improve the effect of seamless ligation and remove disturbance to the seamless ligation by the undigested plasmid background, inverse PCR was performed on the linearized plasmid segment to obtain a final linearized recombinant expression vector pcGAPGA-pepA by taking *Bam*HI single-digested linearized segment as a template and using a primer RpepA F/R, and the result is shown in FIG. 7.

### 4. Ligation of pepF expression cassette with linearized recombinant expression vector pcGAPGA-pepA and transformation of Candida utilis

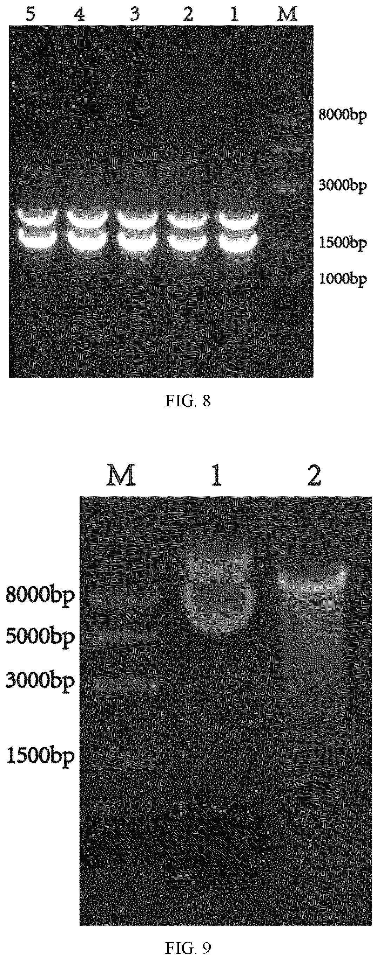

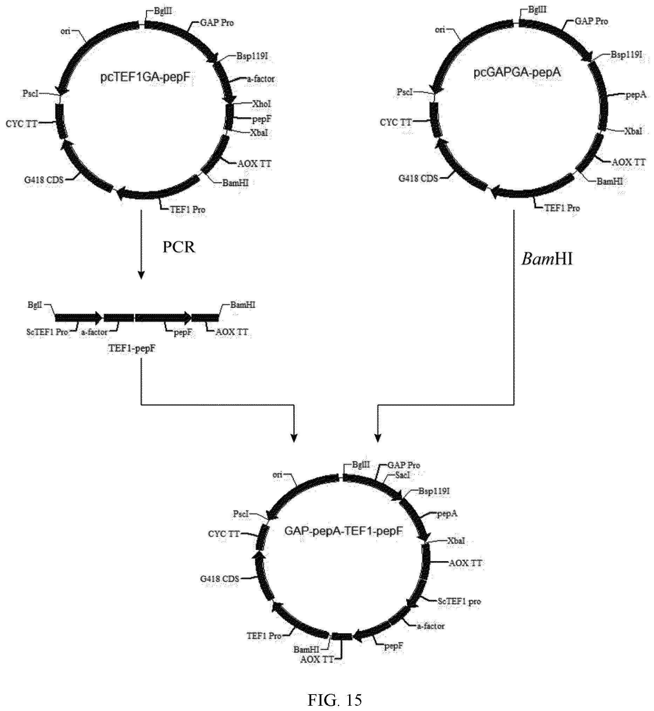

The obtained pepF expression cassette was ligated with the linearized pcGAPGA-pepA recombinant plasmid using a seamless ligation kit to obtain a recombinant dual gene expression vector GAP-pepA-TEF1-pepF, a construction process of which is shown in FIG. 15; then DH5a E. coli was transformed, positive identification of DH5a was performed using primers pepA F/R and pepF F1/R4, and the result is shown in FIG. 8.

Plasmids were extracted from DH5a E. coli containing the double gene recombinant expression vector GAP-pepA-TEF1-pepF plasmid, a Candida utilis TGCE containing endoprotease pepA and carboxypeptidase pepF was obtained after linearized treatment using the restriction endonuclease *Sac*I and transformation of the Candida utilis, the genome of Candida utilis TGCE was identified, and the result is shown in FIG. 9.

### 5. Growth curves for double gene recombinant Candida utilis TGCE protease activity

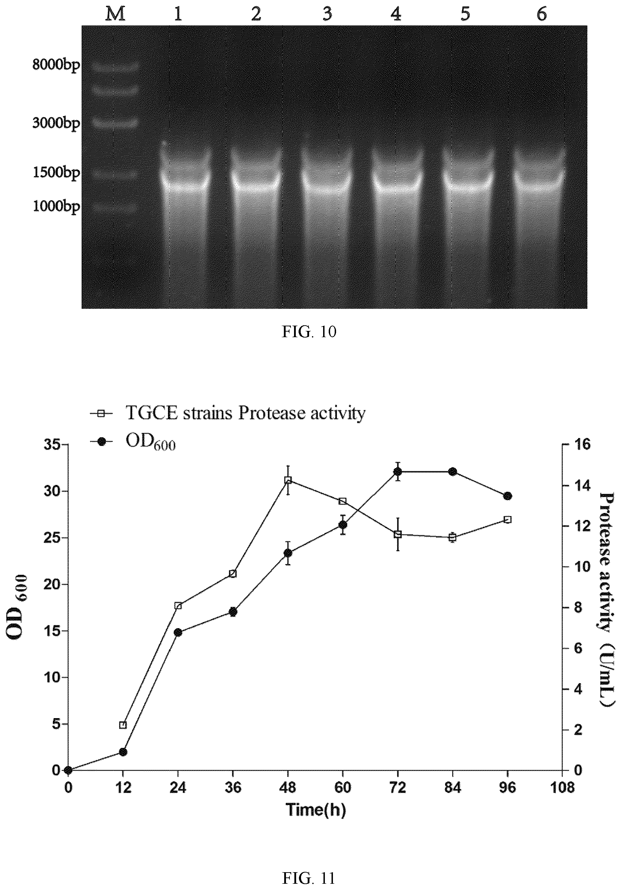

The recombined Candida utilis TGCE containing double genes of endoprotease and carboxypeptidase were cultured and inoculated in 100 mL of YPD culture medium with an initial inoculum size of OD = 0.05, supernatant liquid was removed every 12 hours, the biomass in recombinant Candida utilis and the protease activity in the supernatant liquid were determined using a Folin method to drawn a graph, and the result is shown in FIG. 11.

Referring to FIG. 11, it can be seen that in the growth curve for double gene Candida utilis protease activity, the OD value peaked and exceeded 30 at the 72th hour and was still above 25 for a period of time until the 94th hour, while the protease activity peaked and exceeded 14U/mL at the 48th hour and still fluctuated within an interval of 11 to 14U/mL between the 48th hour to the 96th hour. As to FIG. 13, that is, a growth curve for pepF single gene-containing Candida utilis protease activity, although the OD value thereof peaked and exceeded 30 at the 84th hour, a more obvious drop occurs upon reaching the peak value. The protease activity peaked only below 13U/mL, and a significant drop occurs upon reaching the peak value and dropped below 11U/mL at the 96th hour. Therefore, double gene has a better growth amount, and stable and higher protease activity as compared to the pepF single gene. As to FIG. 14, although the protease activity of the pepA single gene Candida utilis fluctuated within an interval of 14 to 16U/mL after the 72th hour with high protease activity, the growth amount thereof obviously changed after the 60th hour; the high protease activity failed to produce enzyme for a longer time as growth conditions deteriorated, and thus the decrease in the amount of enzyme still leaded to unfavorable effect of enzymatic degradation. However, referring to the double gene represented in FIG. 11, although the protease activity decreased after the 48th hour, the protease activity was still at a higher level, and the growth amount remains at a higher level for a longer period of time, in which a balanced growth condition and protease activity was beneficial to significantly improve the degradation effect. As to FIG. 17, that is, a growth curve for triple-gene Candida utilis protease activity, although the protease activity thereof reached a peak value similar to that of the double gene in the present application at the 48th hour and sustained protease activity with the peak value for a period of time, the OD value thereof smoothly rose to peak and then dropped while the peak value was below 25. Therefore, it is evident that the triple gene is slow in growth rate, and that the growth amount is obviously fewer than the other single-gene or double-gene recombinant *Candida utilis.* Therefore, compared to single gene and triple gene, the double gene in the present application has stable and higher protease activity and growth amount, which balances strain enzyme activity and strain growth conditions, significantly increases degradation effects by unit strains, and is suitable for industrial applications.

In the embodiments described above, the primers used are shown in Table 1, in which the letter F represents a forward primer, R represents a reverse primer, and the sequence numbers represent different primer combinations.

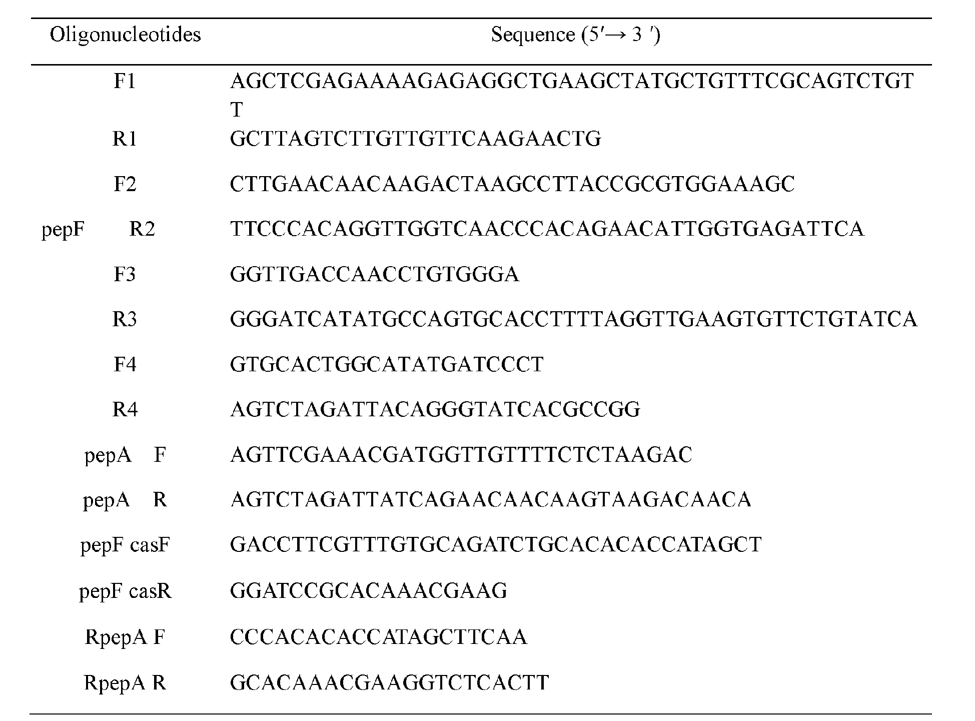

ACGCCGG
Table 1

Obviously, the above embodiments of the present invention are merely examples for clear illustration of the technical solutions of the present invention, and are not intended to limit the implementation of the present invention. Any modification, equivalent substitution, improvement or the like within the spirit and principle of claims of the present invention should be included in the scope of the claims of the present invention.

## Claims

1. A *Candida utilis* double gene co-expression strain for hydrolyzing a protein component in kitchen waste, wherein the *Candida utilis* double gene co-expression strain is constructed by integrating a carboxypeptidase and an endoprotease gene through a *Candida utilis* expression vector onto a *Candida utilis* genome.

2. The *Candida utilis* double gene co-expression strain for hydrolyzing a protein component in kitchen waste according to claim 1, wherein the *Candida utilis* expression vector is a *Candida utilis* polygenic co-expression vector.

3. The *Candida utilis* double gene co-expression strain for hydrolyzing a protein component in kitchen waste according to claim 1, wherein the Candida utilis expression vector is GAP-pepA-TEF1-pepF.

4. A construction method of the *Candida utilis* double gene co-expression strain for hydrolyzing protein components in kitchen waste according to any one of claims 1 to 3, comprising steps of:
S1: ligating an endoprotease gene pepA to an expression vector pcGAPGA;
S2: ligating a carboxypeptidase gene pepF to an expression vector pcTEF1GA;
S3: obtaining a carboxypeptidase gene pepF expression cassette;
S4: linearizing an expression vector pcGAPGA-pepA;
S5: ligating the above carboxypeptidase gene expression cassette to an expression vector pcGAPGA-pepA to obtain a co-expression vector containing both carboxypeptidase and endoprotease gene; and
S6: cutting and linearizing the recombinant double gene co-expression vector obtained from the above construction by restriction endonuclease *Sac*I, transforming the *Candida utilis,* and constructing a genetic recombinant *Candida utilis* double gene co-expression strain.

5. The construction method of the *Candida utilis* double gene co-expression strain for hydrolyzing protein components in kitchen waste according to claim 4, wherein the co-expression vector containing both carboxypeptidase and endoprotease gene in step S5 is GAP-pepA- TEF1-pepF.

6. The construction method of the *Candida utilis* double gene co-expression strain for hydrolyzing protein components in kitchen waste according to claim 4, further comprising: hydrolyzing protein components in kitchen waste by the recombinant *Candida utilis* double gene co-expression strain.

7. The construction method of the *Candida utilis* double gene co-expression strain for hydrolyzing protein components in kitchen waste according to claim 4, wherein the transformation in step S6 comprises a transformation using an electrotransformation method, a freezing method, or a chemical reagent method.

8. The construction method of the *Candida utilis* double gene co-expression strain for hydrolyzing protein components in kitchen waste according to claim 4, wherein the endoprotease in step S1 is endoprotease pepA in Aspergillus niger, and the carboxypeptidase in step S2 is carboxypeptidase pepF in Aspergillus niger.


SEQUENCE LISTING

<110>  Guangdong Recyclean Low-Carbon Technology Co., LTD.

<120>  Candida Utilis Double Gene Co-Expression Strain for Hydrolyzing
Protein Components in Kitchen Waste and Construction Method Thereof

<130>

<160>  2

<170>  PatentIn version 3.5

<210>  1
<211>  1608
<212>  DNA
<213>  Aspergillus niger

<400>  1
ctcgagatgc tgtttcgcag tctgttgtcg acggctgtcc tagccgtctc gctgtgcacg      60

gataatgctt cagctgctaa acatggtcga tttggccaaa aagctcgcga cgccatgaac     120

atcgcgaagc gttccgctaa cgccgtgaaa cactcgttga agatccctgt cgaggactat     180

cagttcttga acaacaagac taagccttac cgcgtggaaa gcctgcctga tgttcacttc     240

gatctgggcg agatgtattc cggcttggtc cctattgaga agggcaacgt gtcacggtcc     300

cttttctttg tcttccagcc cactattggc gagcctgtgg atgagatcac catctggctg     360

aatggtggcc ctggttgcag ttcccttgag gcctttctcc aggagaatgg tagattcgtg     420

tggcagcctg gaacctacca gcctgttgag aacccatact cgtgggtgaa tctcaccaat     480

gttctgtggg ttgaccaacc tgtgggaacg ggattctctc tgggtgtccc aaccgctacg     540

tccgaggagg agattgctga agactttgtg aagttcttca agaactggca gcagatcttt     600

gggatcaaaa acttcaagat ctatgttact ggagaaagtt atgcgggccg ttatgttcct     660

tacatatccg ctgctttcct agatcagaat gatacagaac acttcaacct aaaaggtgca     720

ctggcatatg atccctgtat tggtcagttt gactacgtgc aggaggaagc acctgttgtt     780

ccctttgtcc agaagaacaa tgccctcttc aatttcaatg caagcttttt ggcggaacta     840

gagagcatcc atgagcaatg tggatacaag gatttcatcg accagtatct agtcttccca     900

gcatccggtg tccagccgcc aaaggctatg aactggagcg atcccacctg tgatgtttat     960

gacatcgtta ataacgccgt cctggatccc aacccgtgct tcaaccccta cgaaatcaac    1020

gagatgtgcc ccattctctg ggacgttctt ggattcccca ccgaagtcga ctatctccct    1080

gcgggcgcca gcatctactt tgaccgcgct gatgttaagc gtgccatgca cgctcctaac    1140

atcacctggt ccgagtgctc ggtggagagc gtctttgtcg ggggcgacgg cggtcccgag    1200

caggagggcg actactcggc caaccccatc gagcatgtct tgccccaggt catcgaaggc    1260

accaaccgag ttctgatcgg taacggtgat tatgacatgg tcatccttac caacggcacc    1320

cttctctcga tccagaacat gacatggaat ggaaagcttg gattcgacac ggcccccagc 1380

acccccatca acatcgacat ccctgacctg atgtacaatg aagtgttcat tgagaacggc 1440

tatgacccac aaggtggtca gggtgtcatg ggcatccagc actatgagcg tggtcttatg 1500

tgggctgaga ccttccagag cggacacatg cagccccaat tccaacccag agtgtcatac 1560

cgtcaccttg agtggctgct tggccggcgt gataccctgt aatctaga 1608

<210> 2
<211> 2178
<212> DNA
<213> Aspergillus niger

<400> 2
ttcgaaacga tggttgtttt ctctaagacc gctgctttgg ttttgggttt gtcttctgct 60

gtttctgctg ctccagctcc aaccagaaag ggtttcacca tcaaccaaat cgctagacca 120

gctaacaaga ccagaaccat caacttgcca ggtatgtacg ctagatcttt ggctaagttc 180

ggtggtaccg ttccacaatc tgttaaggag gctgcttcta agggttctgc tgttaccacc 240

ccacaaaaca acgatgagga gtacttgacc ccagttaccg ttggtaagtc taccttgcac 300

ttggatttcg ataccggttc tgctgatttg tgggttttct ctgatgagtt gccatcttct 360

gagcaaaccg gtcacgattt gtacacccca tcttcttctg ctaccaagtt gtctggttac 420

acctgggata tctcttacgg tgatggttct tctgcttctg gtgatgttta cagagatacc 480

gttaccgttg gtggtgttac caccaacaag caagctgttg aggctgcttc taagatctct 540

tctgagttcg ttcaaaacac cgctaacgat ggtttgttgg gtttggcttt ctcttctatc 600

aacaccgttc aaccaaaggc tcaaaccacc ttcttcgata ccgttaagtc tcaattggat 660

tctccattgt tcgctgttca attgaagcac gatgctccag gtgtttacga tttcggttac 720

atcgatgatt ctaagtacac cggttctatc acctacaccg atgctgattc ttctcaaggt 780

tactggggtt tctctaccga tggttactct atcggtgatg gttcttcttc ttcttctggt 840

ttctctgcta tcgctgatac cggtaccacc ttgatcttgt tggatgatga gatcgtttct 900

gcttactacg agcaagtttc tggtgctcaa gagtctgagg aggctggtgg ttacgttttc 960

tcttgttcta ccaacccacc agatttcacc gttgttatcg gtgattacaa ggctgttgtt 1020

ccaggtaagt acatcaacta cgctccaatc tctaccggtt cttctacctg tttcggtggt 1080

atccaatcta actctggttt gggtttgtct atcttgggtg atgttttctt gaagtctcaa 1140

tacgttgttt tcaactctga gggtccaaag ttgggtttcg ctgctcaagc tggtggtggt 1200

ggctcgagcg ctaagtcttc tttcatctct accaccacca ccgatttgac ctctatcaac 1260

acctctgctt actctaccgg ttctatctct accgttgaga ccggtaacag aaccacctct 1320

gaggttatct ctcacgttgt taccacctct accaagttgt ctccaaccgc taccacctct 1380

ttgaccatcg ctcaaacctc tatctactct accgattcta acatcaccgt tggtaccgat 1440

atccacacca cctctgaggt tatctctgat gttgagacca tctccagaga gaccgcttct 1500

accgttgttg ctgctccaac ctctaccacc ggttggaccg gtgctatgaa cacctacatc 1560

tctcaattca cctcttcttc tttcgctacc atcaactcta ccccaatcat ctcttcttct 1620

gctgttttcg agacctctga tgcttctatc gttaacgttc acaccgagaa catcaccaac 1680

accgctgctg ttccatctga ggagccaacc ttcgttaacg ctaccagaaa ctctttgaac 1740

tctttctgtt cttctaagca accatcttct ccatcttctt acacctcttc tccattggtt 1800

tcttctttgt ctgtttctaa gaccttgttg tctacctctt tcaccccatc tgttccaacc 1860

tctaacacct acatcaagac caagaacacc ggttacttcg agcacaccgc tttgaccacc 1920

tcttctgttg gtttgaactc tttctctgag accgctgttt cttctcaagg taccaagatc 1980

gataccttct tggtttcttc tttgatcgct tacccatctt ctgcttctgg ttctcaattg 2040

tctggtatcc aacaaaactt cacctctacc tctttgatga tctctaccta cgagggtaag 2100

gcttctatct tcttctctgc tgagttgggt tctatcatct tcttgttgtt gtcttacttg 2160

ttgttctgat aatctaga 2178